# EUROPÄISCHE PATENTSCHRIFT

(11) **EP 3 028 729 B1**
(45) Veröffentlichungstag und Bekanntmachung des Hinweises auf die Patenterteilung: **16.09.2020**
(21) Anmeldenummer: 15175696.2
(22) Anmeldetag: 07.07.2015
(51) Int. Cl.: A61M 5/19, A61M 5/31, A61M 5/178

(54) **INJEKTOR ZUR ABGABE FLÜSSIGEN ODER PASTÖSEN STOFFS, INSBESONDERE MEDIKAMENT**
INJECTOR FOR DISPENSING A LIQUID OR PASTE SUBSTANCE, IN PARTICULAR A MEDICAMENT
INJECTEUR DE DISTRIBUTION DE MATIERES LIQUIDES OU PATEUSES, EN PARTICULIER DES MEDICAMENTS

(30) Priorität: 05.12.2014 DE 102014018077; 19.02.2015 DE 102015002234
(43) Veröffentlichungstag der Anmeldung: 08.06.2016
(73) Patentinhaber: elm-plastic GmbH, 54647 Dudeldorf (DE)
(72) Erfinder: Lonien, Birgit, 54647 Dudeldorf (DE); Möhs, Sascha, 54647 Dudeldorf (DE)
(74) Vertreter: Zinnecker, Armin

(56) Entgegenhaltungen:
- EP-A1- 0 654 280
- EP-A1- 1 093 826
- EP-A2- 2 168 619
- WO-A1-2013/021186
- DE-A1-102013 112 521
- US-A- 3 659 749

## Beschreibung

Die Erfindung betrifft einen Injektor zur Abgabe eines ersten oder eines zweiten flüssigen oder pastösen Stoffs. Bei dem ersten und/oder zweiten flüssigen oder pastösen Stoff kann es sich insbesondere um ein Medikament handeln oder um einen Stoff, der eines oder mehrere Medikamente enthält.

Der Injektor umfasst einen äußeren Injektorkörper, einen inneren Injektorkörper und einen Stößel. In den äußeren Injektorkörper wird ein erstes Volumen für den ersten flüssigen oder pastösen Stoff gebildet. In dem inneren Injektorkörper wird ein zweites Volumen für den zweiten flüssigen oder pastösen Stoff gebildet. Der innere Injektorkörper ist in dem äußeren Injektorkörper längsverschieblich geführt. Er kann dort drehbar oder nicht drehbar längsverschieblich geführt sein. Der Stößel ist in dem inneren Injektorkörper längsverschieblich geführt. Er kann dort drehbar oder nicht drehbar längsverschieblich geführt sein.

Durch eine Bewegung des Stößels wird zunächst der innere Injektorkörper in dem äußeren Injektorkörper bewegt. Hierdurch wird der erste flüssige oder pastöse Stoff durch eine Öffnung im Bereich der Bodenwand des äußeren Injektorkörpers abgegeben. Wenn der innere Injektorkörper seine Endposition in dem äußeren Injektorkörper erreicht hat, kann der Stößel in dem inneren Injektorkörper bewegt werden. Dadurch kann der zweite flüssige oder pastöse Stoff abgegeben werden.

Aus der DD 210 386 B ist ein Injektor zur Abgabe eines ersten und eines zweiten flüssigen oder pastösen Stoffs mit einem äußeren Injektorkörper, in dem ein innerer Injektorkörper längsverschieblich geführt ist, in dem ein Stößel längsverschieblich geführt ist, bekannt.

Die US 2 157 503 A offenbart eine Spritze, in die eine mit Flüssigkeit gefüllte Ampulle eingebracht werden kann. Mit der Flüssigkeit der Ampulle kann eine Tablette, die ein medizinisches Präparat enthält, vermischt werden.

Aus der DE 695 31 774 T2 ist ein Blutentnahme- und Blutisolationssystem bekannt, das einen Spritzenzylinder, einen Hauptkolben und einen Teilerkolben umfasst.

Die US 3 659 749 A offenbart eine Vorrichtung zum Mischen eines flüssigen Stoffes mit einem pulverförmigen Stoff.

Aus der EP 1 093 826 A1 ist eine Vorrichtung zum Mischen eines ersten und eines zweiten flüssigen Stoffes bekannt.

EP 654 280 A1 zeigt eine Einwegspritze.

EP 2 168 619 A2 zeigt einen Injektor zur Abgabe eines ersten und eines zweiten flüssigen Stoffes, bei welchem der zweite Stoff in einer Endposition des inneren Injektorkörpers durch einen Seitenkanal zur Abgabetülle fließt.

Die WO 2013/021186 A1 offenbart einen Injektor zur Abgabe eines ersten und eines zweiten flüssigen Stoffes gemäß dem Oberbegriff von Anspruch 1.

Aufgabe der Erfindung ist es, einen verbesserten Injektor der eingangs angegebenen Art vorzuschlagen.

Erfindungsgemäß wird diese Aufgabe durch die Merkmale des Anspruchs 1 gelöst. Der innere Injektorkörper weist eine Manschette auf, die an der Innenwand des äußeren Injektorkörpers dichtend anliegt. Insbesondere liegt die Manschette während der Bewegung des inneren Injektorkörpers in dem äußeren Injektorkörper an der Innenwand des äußeren Injektorkörpers dichtend an. Wenn sich die Manschette in der Endposition in dem äußeren Injektorkörper befindet, ist sie von dem zweiten flüssigen oder pastösen Stoff umströmbar. Dadurch ist gewährleistet, dass nach der Abgabe des ersten flüssigen oder pastösen Stoffs auch der zweite flüssige oder pastöse Stoff abgegeben werden kann. Weiterhin sind in der Bodenwand des inneren Injektorkörpers einer oder mehrere Kanäle vorgesehen.

Vorteilhafte Weiterbildungen der Erfindung sind in den Unteransprüchen beschrieben.

Vorteilhaft ist es, wenn der äußere Injektorkörper in seinem Endbereich einen oder mehrere Kanäle aufweist. Der oder die Kanäle können sich in der Innenwand des Endbereichs des äußeren Injektorkörpers befinden. Der zweite flüssige oder pastöse Stoff kann die Manschette umströmen, wenn sich die Manschette in der Endposition in dem äußeren Injektorkörper befindet, indem der zweite flüssige oder pastöse Stoff den oder die Kanäle durchströmt.

Vorzugsweise weist die Manschette an ihrer Unterseite eine oder mehrere Nuten auf. Der zweite flüssige oder pastöse Stoff kann durch diese Nuten strömen, wenn sich die Manschette in der Endposition in dem äußeren Injektorkörper befindet.

Eine weitere vorteilhafte Weiterbildung ist dadurch gekennzeichnet, dass die Manschette umströmbar deformiert ist, wenn sie sich in der Endposition in dem äußeren Injektorkörper befindet. Durch die Deformation der Manschette können Kanäle gebildet werden, die von dem zweiten flüssigen oder pastösen Stoff durchströmt werden, wenn sich die Manschette in der Endposition in dem äußeren Injektorkörper befindet.

Vorteilhaft ist es, wenn im Endbereich des äußeren Injektorkörpers Stege vorgesehen sind. Die Stege sind vorzugsweise in der Bodenwand des äußeren Injektorkörpers vorgesehen. Durch die Stege kann die Manschette deformiert werden. Der zweite flüssige oder pastöse Stoff kann die Bereiche zwischen den Stegen durchströmen. Vorteilhaft ist es, wenn mehr als zwei Stege vorhanden sind. Die Stege sind vorzugsweise gleichmäßig über den Umfang verteilt.

Der erfindungsgemäße Injektor kann vorzugsweise zur Verabreichung einer oder mehrerer Flüssigkeiten und/oder einer oder/mehrerer Pasten und/oder eines oder mehrerer Medikamente in ein Euter eines Tiers verwendet werden. Er kann also insbesondere als Euterinjektor eingesetzt werden.

Vorteilhaft ist es, wenn die Manschette mit dem inneren Injektorkörper lösbar verbunden ist.

Es kann allerdings auch vorteilhaft sein, wenn die Manschette mit dem inneren Injektorkörper einstückig ausgebildet ist.

Der oder die Kanäle können zumindest teilweise in radialer Richtung verlaufen.

Ausführungsbeispiele der Erfindung werden nachstehend anhand der beigefügten Zeichnung im Einzelnen erläutert. In der Zeichnung zeigt
- Fig. 1: eine erste Ausführungsform der Erfindung in der Ausgangsstellung in einer seitlichen Schnittansicht,
- Fig. 2: den Gegenstand der Fig. 1 nach der Abgabe des ersten flüssigen oder pastösen Stoffs,
- Fig. 3: den Gegenstand der Fig. 1 und 2 nach der Abgabe des zweiten flüssigen oder pastösen Stoffs,
- Fig. 4: eine vergrößerte Einzelheit aus der Fig. 3,
- Fig. 5: eine zweite Ausführungsform der Erfindung in der Ausgangsstellung in einer seitlichen Schnittansicht,
- Fig. 6: den Gegenstand der Fig. 5 nach der Abgabe des ersten flüssigen oder pastösen Stoffs,
- Fig. 7: den Gegenstand der Fig. 5 und 6 nach der Abgabe des zweiten flüssigen oder pastösen Stoffs,
- Fig. 8: eine vergrößerte Einzelheit aus der Fig. 7,
- Fig. 9: eine abgewandelte Ausführungsform der ersten Ausführungsform der Erfindung, bei der die Manschette mit dem inneren Injektorkörper einstückig ausgebildet ist,
- Fig. 10: eine vergrößerte Einzelheit aus der Fig. 9 und
- Fig. 11: eine Abwandlung der Ausführungsform nach Fig. 9 und 10, bei der die Kanäle in dem inneren Injektorkörper teilweise in radialer Richtung verlaufen.

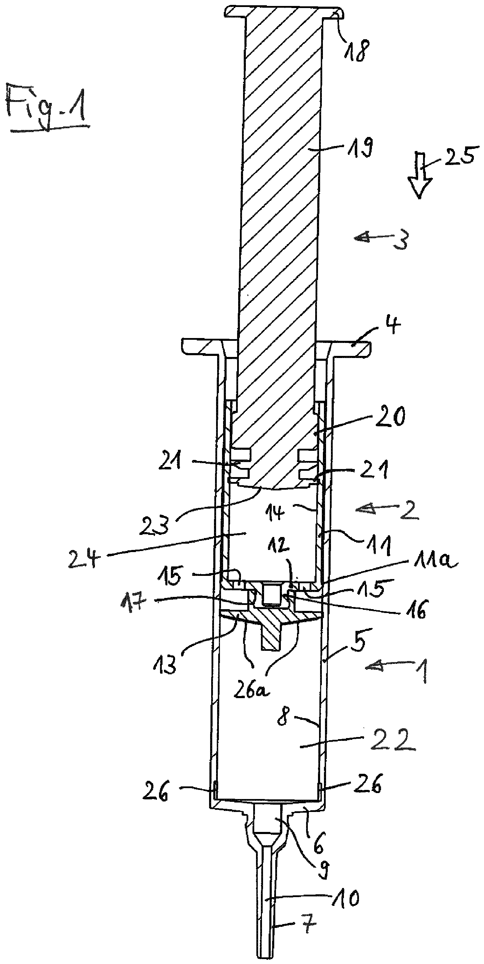

Die in Fig. 1 bis 4 gezeigte erste Ausführungsform des erfindungsgemäßen Injektors umfasst einen äußeren Injektorkörper 1, einen inneren Injektorkörper 2 und einen Stößel 3. Der äußere Injektorkörper 1 umfasst einen oberen Rand 4, der nach außen gerichtet ist, ein Mantelteil 5, eine Bodenwand 6 und eine Abgabetülle 7. Das Mantelteil 5 ist zylinderförmig ausgebildet. Es weist eine Innenwand 8 auf, längs der der innere Injektorkörper 2 längsverschieblich geführt ist. Die Bodenwand 6 weist in ihrer Mitte eine Abgabeöffnung 9 auf, die mit einem Abgabekanal 10 in der Abgabetülle 7 verbunden ist. Der äußere Injektorkörper 1 ist einstückig ausgebildet.

Der innere Injektorkörper 2 umfasst ein Mantelteil 11, eine Bodenwand 12 und eine Manschette 13. Das Mantelteil 11 ist zylinderförmig ausgebildet. Seine Außenwand ist längs der Innenwand 8 des Mantelteils 5 des äußeren Injektorkörpers 1 längsverschieblich geführt. Hierfür weist das Mantelteil 11 an seinem unteren Ende eine Dichtfläche 11a auf, deren Durchmesser größer ist als der sonstige Durchmesser des Mantelteils 11. Die Dichtfläche 11a liegt an der Innenwand 8 an. Die Hauptfunktion der Dichtfläche 11a besteht in der Gewährleistung einer Dichtung. Darüber hinaus erfüllt sie auch einer Führungsfunktion.

Das Mantelteil 11 weist eine Innenwand 14 auf, in der der Stößel 3 längsverschieblich geführt ist. In der Bodenwand 12 des inneren Injektorkörpers 2 sind Kanäle 15 vorhanden. Die Kanäle 15 sind gleichmäßig über den Umfang verteilt.

Die Bodenwand 12 weist an ihrer Unterseite, die der Bodenwand 6 des äußeren Injektorkörpers 1 zugewandt ist, einen Ansatz 16 auf, auf den die Manschette 13 aufgesteckt ist. Die Manschette 13 ist auf diese Weise lösbar mit dem Ansatz 16 verbunden. Zur besseren Befestigung weist der Ansatz 16 in seinem unteren Endbereich, der der Bodenwand 6 des äußeren Injektorkörpers 1 zugewandt ist, eine im Durchmesser größere Wulst 17 auf. Die Wulst 17 ist nach außen abgerundet. Das Mantelteil 11, die Bodenwand 12 und der Ansatz 16 des inneren Injektorkörpers 2 sind einstückig ausgebildet.

Der Stößel 3 umfasst einen oberen Rand 18, der nach außen gerichtet ist, einen Stößelkörper 19, eine Führungsfläche 20 und Dichtungen 21. Der Stößelkörper 19 weit einen kreuzförmigen Querschnitt auf. Er kann allerdings auch auf andere Weise ausgestaltet, insbesondere zylinderförmig ausgebildet sein. Der Durchmesser der Innenwand 14 des Mantelteils 11 des inneren Injektorkörpers 2 ist größer als der Außendurchmesser des Stößelkörpers 19. Die Führungsfläche 20 des Stößels 3 liegt an der Innenwand 14 des Mantelteils 11 des inneren Injektorkörpers 2 an. Sie wird an der Innenwand 14 geführt. Die Dichtungen 21 liegen dichtend an der Innenwand 14 des Mantelteils 11 des inneren Injektorkörpers 2 an. Die untere Dichtung 21, die der Bodenwand 12 des inneren Injektorkörpers 2 zugewandt ist, weist einen größeren Durchmesser als die obere Dichtung 21 auf, die dem oberen Rand 18 des Stößels 3 zugewandt ist. Die untere Dichtung 21 bildet die Hauptdichtung, die obere Dichtung 21 bildet eine Zusatzdichtung.

In Betrieb befindet sich der Injektor zunächst in der in Fig. 1 gezeigten Stellung. Die Manschette 13 des inneren Injektorkörpers 2 befindet sich im Abstand von der Bodenwand 6 des äußeren Injektorkörpers 1. Hierdurch wird ein erstes Volumen 22 für den ersten flüssigen oder pastösen Stoff gebildet. Die untere Endfläche 23 des Stößels 3 befindet sich im Abstand von der Bodenwand 12 des inneren Injektorkörpers 2. Hierdurch wird ein zweites Volumen 24 für den zweiten flüssigen oder pastösen Stoff gebildet.

Zu Beginn des Abgabevorgangs wird der Stößel 3 in Richtung des Pfeils 25 nach unten bewegt. Dabei werden der Stößel 3 und der innere Injektorkörper 2 gegenüber dem äußeren Injektorkörper 1 gemeinsam nach unten bewegt. Das Volumen 24, die Kanäle 25 und der Ringraum zwischen der Bodenwand 12 des inneren Injektorkörpers 2 und der Manschette 13 bilden einen insgesamt abgedichteten Raum, der durch die Dichtungen 21 und die Manschette 13 abgedichtet wird. Durch diesen insgesamt abgedichteten Raum ist gewährleistet, dass zunächst die aus Stößel 3 und innerem Injektorkörper 2 gebildete Einheit gegenüber dem äußeren Injektorkörper 1 nach unten bewegt wird.

Der Stößel 3 und der innere Injektorkörper 2 werden also gegenüber dem äußeren Injektorkörper 1 gemeinsam nach unten bewegt. Hierdurch bewegt sich die Manschette 13 auf die Bodenwand 6 des äußeren Injektorkörpers 1 zu, wodurch das erste Volumen 22 verkleinert und der darin befindliche erste flüssige oder pastöse Stoff durch die Abgabeöffnung 9 und den Abgabekanal 10 herausgedrückt wird.

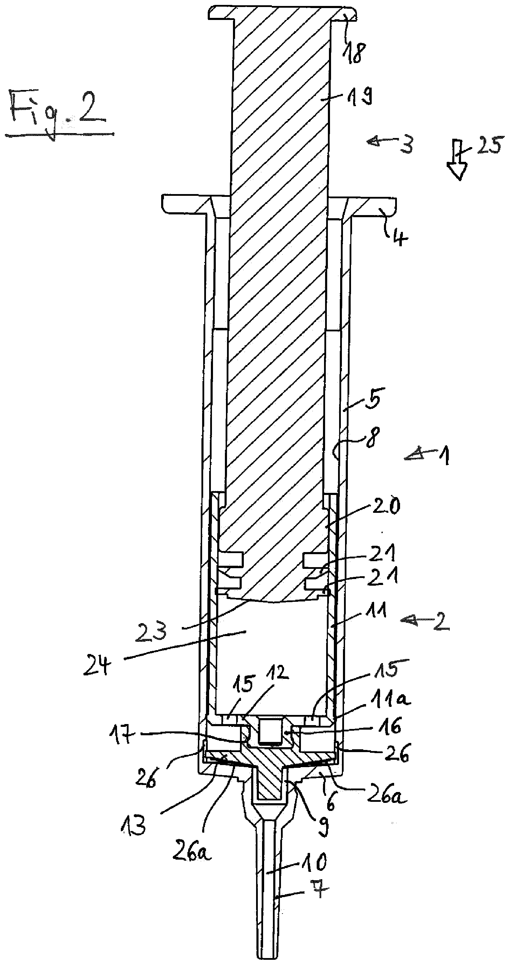

Dieser Vorgang wird durchgeführt, bis die in Fig. 2 gezeigte Stellung erreicht ist, in der sich die Manschette 13 in der Endposition in dem äußeren Injektorkörper 2 befindet. In dieser unteren Endposition liegt die Manschette 13 an der Bodenwand 6 des äußeren Injektorkörpers 1 an.

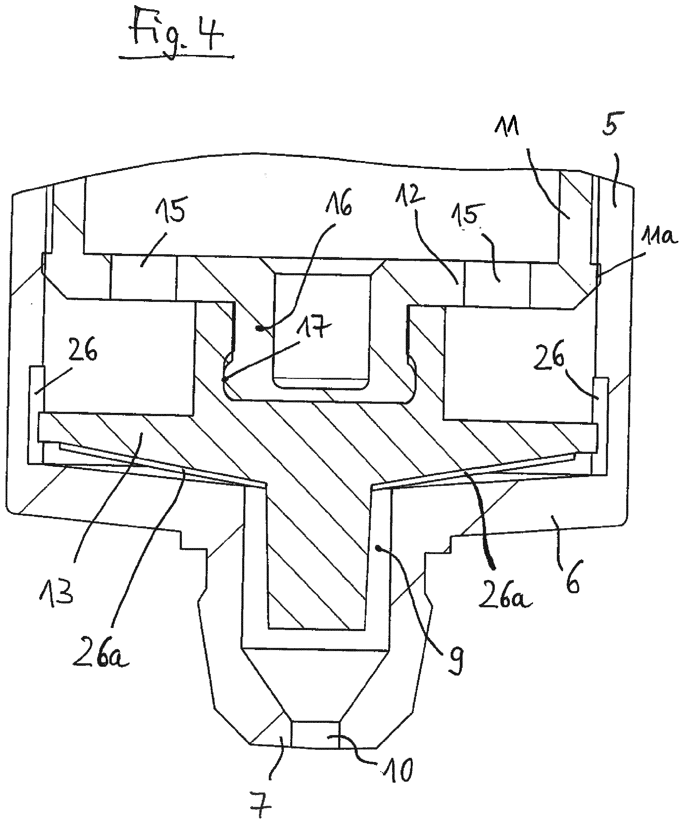

Der äußere Injektorkörper 1 weist in seinem Endbereich mehrere Kanäle 26 auf. Die Kanäle 26 befinden sich in der Innenwand 8 des Mantelteils 5 des äußeren Injektorkörpers 1. Sie beginnen an der Bodenwand 6 des äußeren Injektorkörpers 1 und weisen von dort nach oben. Sie sind so lang ausgebildet, dass sie die obere Endfläche der Manschette 13 überragen, wenn sich die Manschette 13 in ihrer in Fig. 2 und 4 gezeigten Endposition befindet. Ferner ist die Tiefe der Kanäle 26 derart bemessen, dass sie in Umfangsrichtung weiter außen liegt als der Außendurchmesser der Manschette 13. Auf diese Weise bilden die Kanäle 26 einen Weg zur Umströmung der Manschette 13. Die Kanäle 26 sind gleichmäßig über den Umfang des Mantelteils 5 des äußeren Injektorkörpers 1 verteilt.

Die Manschette 13 weist an ihrer Unterseite mehrere Nuten 26a auf. Die Nuten 26a verlaufen in radialer Richtung. Sie verlaufen von innen unten nach außen oben. Die Nuten 26a sind gleichmäßig über den Umfang der Manschette 13 verteilt. Durch die Nuten 26a wird verhindert, dass die Unterseite der Manschette 13 vollständig und dichtend auf der oberen Umrandung der Abgabeöffnung 9 aufliegt. Auf diese Weise bilden die Nuten 26a einen Weg zur Umströmung der Manschette 13.

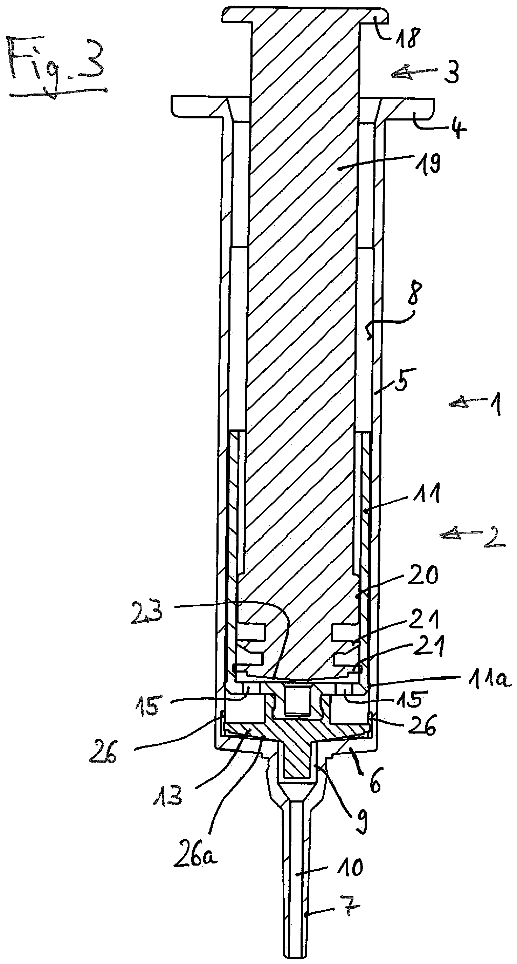

Wenn der Injektor die in Fig. 2 gezeigte Stellung erreicht hat wird der Stößel 3 weiter nach unten bewegt. Seine untere Endfläche 23 bewegt sich auf die Bodenwand 12 des inneren Injektorkörpers 2 zu. Hierdurch wird das zweite Volumen 24 verkleinert. Der darin befindliche zweite flüssige oder pastöse Stoff wird aufgrund dieser Bewegung durch die Kanäle 15 und die Kanäle 26 und die Nuten 26a an der Manschette 13 vorbei in die Abgabeöffnung 9 und den Abgabekanal 10 gedrückt. Die Bewegung des Stößels 3 wird fortgesetzt, bis die in Fig. 3 gezeigte Endstellung erreicht ist, in der die untere Endfläche 23 des Stößels 3 an der Bodenwand 12 des inneren Injektorkörpers 2 anliegt.

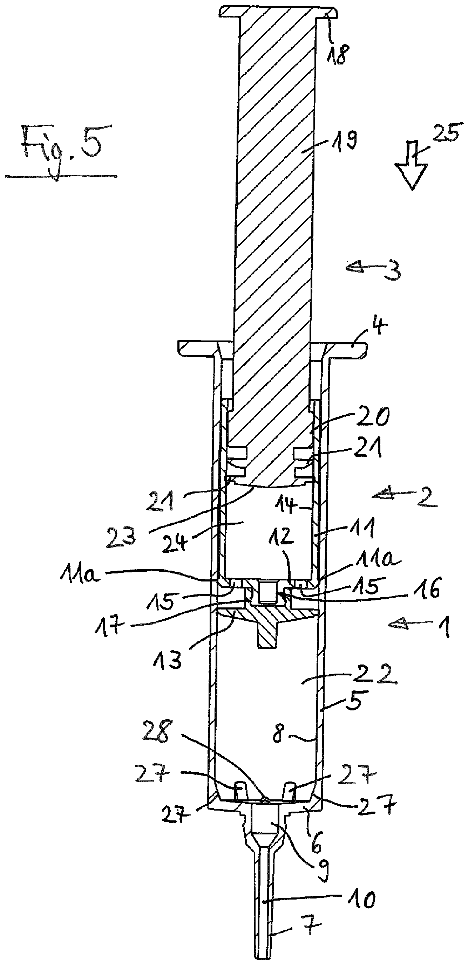

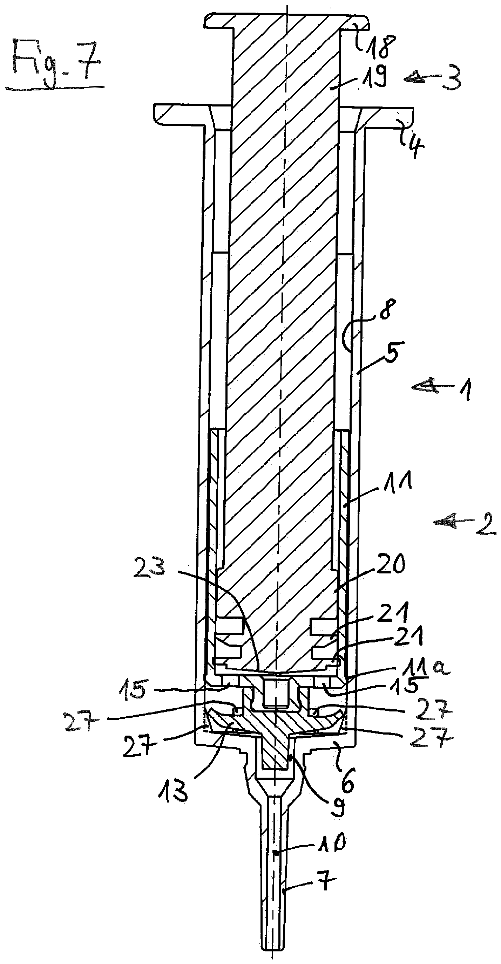

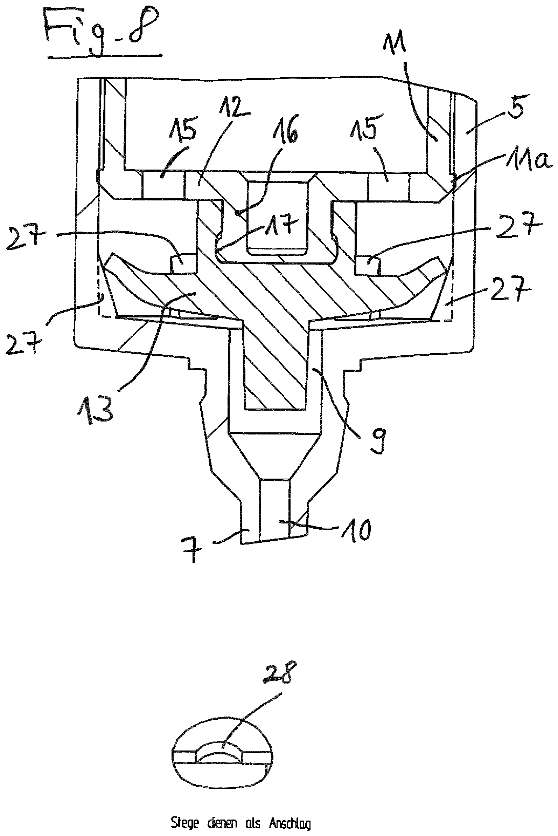

In Fig. 5 bis 8 ist eine zweite Ausführungsform des erfindungsgemäßen Injektors gezeigt, bei der übereinstimmende Bestandteile mit denselben Bezugszeichen versehen sind und nicht erneut beschrieben werden. Hier weist der äußere Injektorkörper 1 in seinem Endbereich Stege 27 auf. Die Stege 27 sind mit der Bodenwand 6 des äußeren Injektorkörpers 1 verbunden. Sie ragen von der Bodenwand 6 nach oben, also zu der Manschette 13 hin. Die Stege 27 sind mit der Bodenwand 6 einstückig verbunden. Sie weisen von unten innen nach außen oben. Die Stege 27 sind gleichmäßig über den Umfang verteilt. Im Ausführungsbeispiel sind sechs Stege 27 vorhanden.

Zwischen zwei Stegen 27 sind Erhebungen 28 vorhanden. Sie weisen nach oben, also zu der Manschette 13 hin. Die Erhebungen 28 befinden sich in der Nähe der Abgabeöffnung 9 in der Bodenwand 6 des äußeren Injektorkörpers 1. Die Erhebungen 28 sind gleichmäßig über den Umfang verteilt. Durch die Erhebungen 28 wird ein Anschlag für die Manschette 13 gebildet.

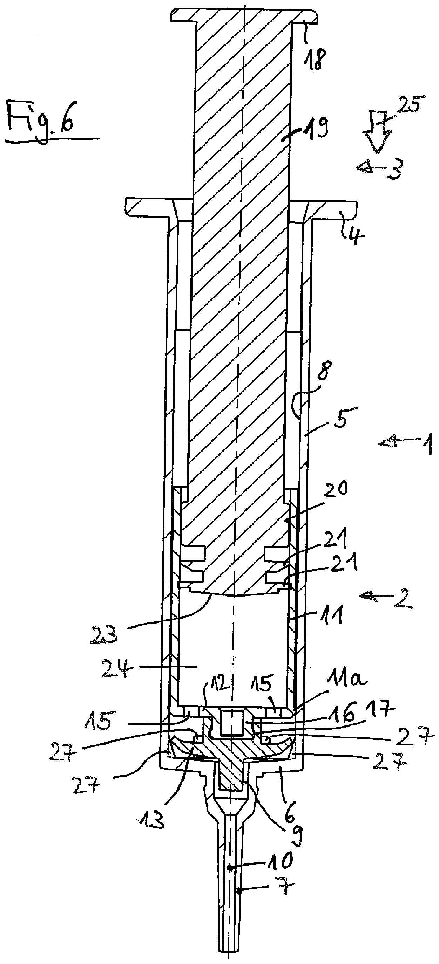

Die Manschette 13 ist deformierbar ausgebildet. Sie ist zumindest in ihrem Außenbereich deformierbar. Wenn die Manschette 13 zu ihrer Endposition hin bewegt wird, die in Fig. 6, 7 und 8 dargestellt ist, wird ihr Außenbereich von den Stegen 27 nach oben verformt, also zu der Bodenwand 12 des inneren Injektorkörpers 2 hin. Dadurch wird der äußere Rand der Manschette 13 von der Innenwand 8 des äußeren Injektorkörpers 1 wegbewegt. Die Manschette 13 liegt dadurch nicht mehr dichtend an der Innenwand 8 an. Sie kann in den Bereichen zwischen den Stegen 27 von dem zweiten flüssigen oder pastösen Stoff umströmt werden.

Der Weg der Manschette 13 zu der Bodenwand 6 hin wird durch die Erhebungen 28 begrenzt, die einen Anschlag für die untere Endfläche der Manschette 13 bilden. Dadurch ist gewährleistet, dass die untere Endfläche der Manschette 13 nicht vollständig an der oberen Umrandung der Abgabeöffnung 9 anliegt, so dass der zweite flüssige oder pastöse Stoff aus dem Bereich der Bodenwand 6 an der unteren Endfläche der Manschette 13 vorbei in die Abgabeöffnung 9 hineinströmen kann.

Durch die Erfindung wird ein 2-Komponenten-Injektor (auch als 2K-Injektor bezeichnet) geschaffen, durch den zwei unterschiedliche Komponenten sequentiell verabreicht werden können. Die beiden unterschiedlichen Komponenten werden separat in dem 2K-Injektor aufbewahrt, nämlich in dessen Volumen 22 und 24. Sie können sich nicht vermischen oder gegenseitig beeinflussen.

Im Betrieb wird der innere Injektorkörper 2 des 2K-Injektors durch Drücken des Stößels 3 im äußeren Injektorkörper 1 bewegt, wodurch die erste Komponente durch den Abgabekanal 10 der Abgabetülle 7 herausgedrückt wird. Wenn die erste Komponente vollständig aus dem äußeren Injektorkörper 1 herausgedrückt worden ist befindet sich der innere Injektorkörper 2 in seiner Endposition in dem äußeren Injektorkörper 1, wie in Fig. 2 und 6 gezeigt. In dieser Endposition wird die Manschette bei der ersten Ausführungsform nach Fig. 1 bis 4 durch Kanäle 26 im äußeren Injektorkörper 1 freigelegt. Die zweite Komponente kann jetzt durch die Kanäle 15 des inneren Injektorkörpers 2 und die Kanäle 26 in dem äußeren Injektorkörper 1 strömen und durch den Abgabekanal 10 in dessen Abgabetülle 7 herausgedrückt werden.

Bei der zweiten Ausführungsform nach Fig. 5 bis 8 wird die Endposition der Manschette 13 durch die Erhebungen 28, die als Stege ausgebildet sein können, im äußeren Injektorkörper 1 festgelegt. Durch die Erhebungen 28 bzw. Stege wird ein entsprechender Freiraum bzw. Kanal zwischen dem äußeren Injektorkörper 1 und der Manschette 13 gebildet. In dieser Endposition wird die Manschette 13 durch Stege 27 im äußeren Injektorkörper 1 so deformiert, dass sie undicht wird. Die zweite Komponente kann dann durch die Kanäle 15 des inneren Injektorkörpers 2 und an der deformierten, undichten Manschette 13 vorbei in den äußeren Injektorkörper 1 gelangen und durch dessen Abgabetülle 7 herausgedrückt werden.

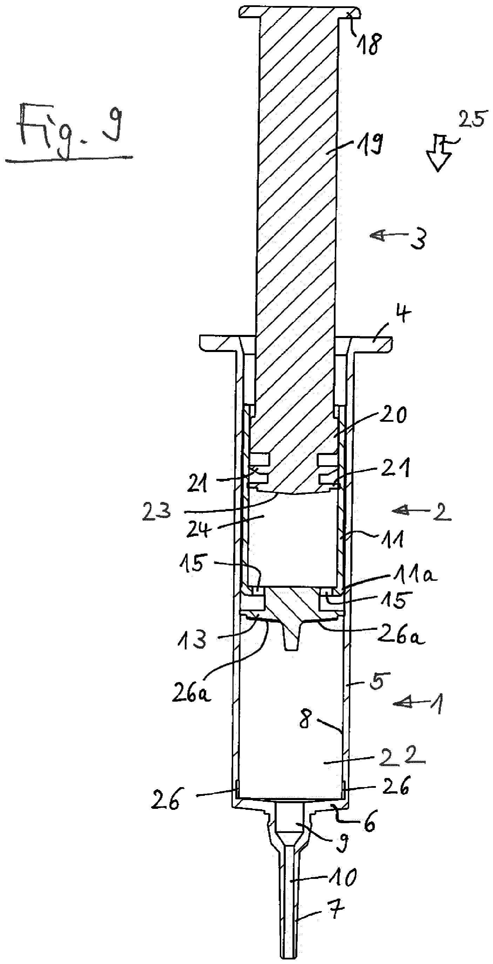

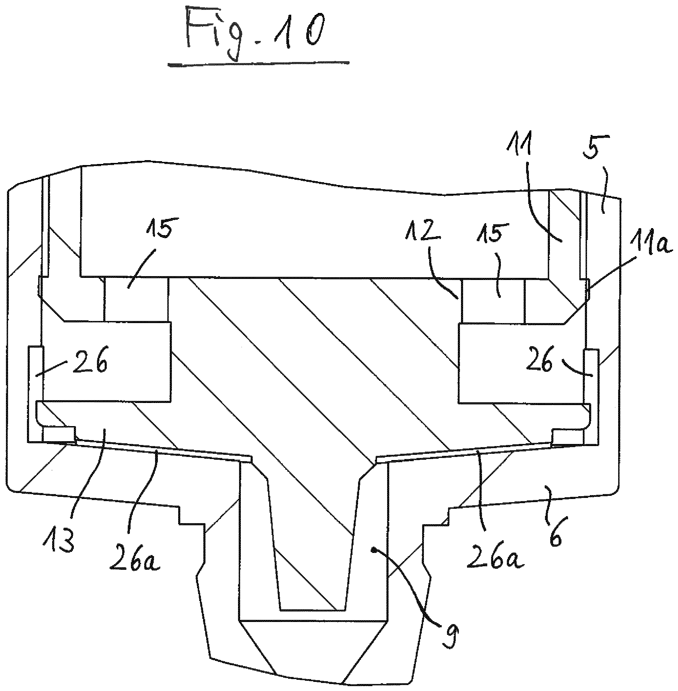

Fig. 9 und 10 zeigen eine Abwandlung der ersten Ausführungsform nach Fig. 1 bis 4, bei der die Manschette 13 mit dem inneren Injektorkörper 2 einstückig ausgebildet ist.

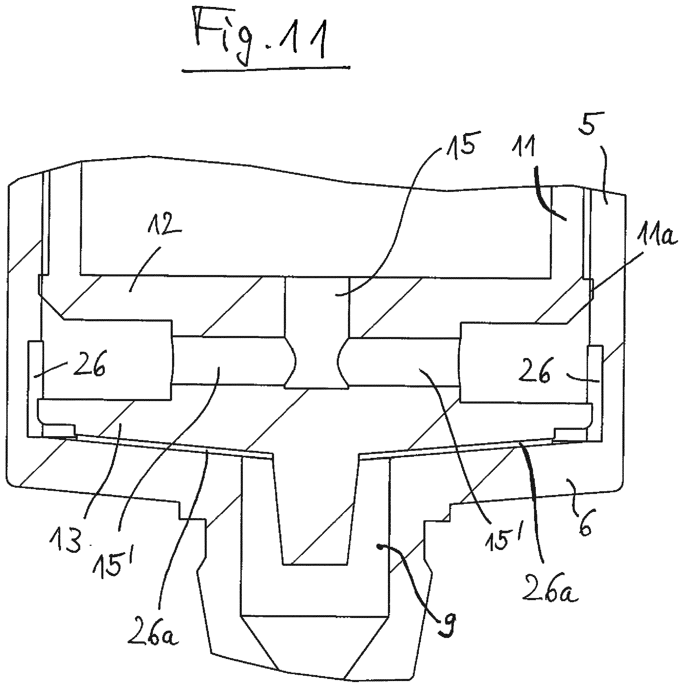

Bei der weiteren Abwandlung nach Fig. 11 ist die Manschette 13 ebenfalls einstückig mit dem inneren Injektorkörper 2 ausgebildet. Hier weisen die Kanäle 15 von dem Volumen 24 zu dem Ringraum zwischen der Bodenwand 12 des inneren Injektorkörpers 2 und der Manschette 13 einen gemeinsamen Abschnitt 15 und radiale Abschnitte 15' auf. Der gemeinsame Abschnitt 15 befindet sich in der Mitte der Bodenwand 12. Er verläuft dort in axialer Richtung nach unten. Die radialen Abschnitte 15' verlaufen von dem unteren Ende des gemeinsamen Abschnitts 15 in radialer Richtung nach außen zu dem erwähnten Ringraum. Die Ausführungsform nach Fig. 11 kann auch dann angewendet werden, wenn die Manschette 13 mit dem inneren Injektorkörper 2 lösbar verbunden ist.

## Patentansprüche

1. Injektor zur Abgabe eines ersten und eines zweiten flüssigen oder pastösen Stoffs, insbesondere eines Medikaments, mit einem äußeren Injektorkörper (1), in dem ein innerer Injektorkörper (2) längsverschieblich geführt ist, in dem ein Stößel (3) längsverschieblich geführt ist, wobei der äußere Injektorkörper ein Mantelteil (5), eine Bodenwand (6) und eine Abgabetülle aufweist, wobei die Bodenwand (6) in der Mitte eine Abgabeöffnung (9) aufweist, die mit einem Abgabekanal (10) in der Abgabetülle verbunden ist, wobei der innere Injektorkörper (2) eine Bodenwand (12) aufweist, in der einer oder mehrerer Kanäle (15) vorgesehen sind, und eine an der Innenwand (8) des äußeren Injektorkörpers (1) dichtend anliegende Manschette (13) aufweist, die von dem zweiten flüssigen oder pastösen Stoff umströmbar ist, wenn sie sich in der Endposition in dem äußeren Injektorkörper (1) befindet, so dass nach der Abgabe des ersten flüssigen oder pastösen Stoffs der zweite flüssige oder pastöse Stoff abgegeben werden kann, dadurch charakterisiert, dass dieser an der Manschette (13) vorbei in die Abgabeöffnung (9) und den Abgabekanal (10) gedrückt wird,

2. Injektor nach Anspruch 1, **dadurch gekennzeichnet, dass** der äußere Injektorkörper (1) in seinem Endbereich einen oder mehrere Kanäle (26) aufweist.

3. Injektor nach Anspruch 1 oder 2, **dadurch gekennzeichnet, dass** die Manschette 13 an ihrer Unterseite eine oder mehrere Nuten (26a) aufweist.

4. Injektor nach einem der vorhergehenden Ansprüche, **dadurch gekennzeichnet, dass** die Manschette (13) umströmbar deformiert ist, wenn sie sich in der Endposition in dem äußeren Injektorkörper (1) befindet.

5. Injektor nach einem der vorhergehenden Ansprüche, **gekennzeichnet durch** Stege (27) im Endbereich des äußeren Injektorkörpers (1).

6. Injektor nach einem der vorhergehenden Ansprüche, **gekennzeichnet durch** eine Erhebung (28) in der Bodenwand (6) des äußeren Injektorkörpers (1).

7. Injektor nach einem der vorhergehenden Ansprüche, **dadurch gekennzeichnet, dass** die Manschette (13) mit dem inneren Injektorkörper (2) lösbar verbunden ist.

8. Injektor nach einem der Ansprüche 1 bis 6, **dadurch gekennzeichnet, dass** die Manschette (13) mit dem inneren Injektorkörper (2) einstückig ausgebildet ist.

9. Injektor nach einem der Ansprüche 1 bis 8, **dadurch gekennzeichnet, dass** der oder die Kanäle (15) in der Bodenwand (12) des inneren Injektorkörpers (2) von dem Volumen (24) des inneren Injektorkörpers (2) zu einem Ringraum zwischen der Bodenwand (12) des inneren Injektorkörpers (2) und der Manschette (13) führen.

10. Injektor nach Anspruch 1 oder 9, **dadurch gekennzeichnet, dass** die Kanäle (15, 15') zumindest teilweise in radialer Richtung verlaufen.

## Claims

1. An injector for administering a first and a second liquid or pasty substance, in particular a drug, comprising an outer injector body (1) in which an inner injector body (2) is longitudinally displaceably guided in which a plunger (3) is longitudinally displaceably guided, wherein the outer injector body comprises a jacket part (5), a base wall (6) and an administration spout, wherein the base wall (6) comprises an administration opening (9) at its center, which is connected to an administration channel (10) in the administration spout, wherein the inner injector body (2) comprises a base wall (12) in which one or multiple channels (15) are provided, and a cuff (13) which sealingly contacts the inner wall (8) of the outer injector body (1) and which can be flowed around by the second liquid or pasty substance when it is in the end position in the outer injector body (1), so that after administration of the first liquid or pasty substance, the second liquid or pasty substance can be administered, **characterized in that** this substance is pressed passed the cuff (13) into the administration opening (9) and the administration channel (10).

2. The injector in accordance with claim 1, **characterized in that** the outer injector body (1) has one or more channels (26) in its end region.

3. The injector in accordance with claim 1 or 2, **characterized in that** the cuff 13 has one or more grooves (26a) at its lower side.

4. The injector in accordance with one of the preceding claims, **characterized in that** the cuff (13) is deformed in a manner such that it can be flowed around when it is in the end position in the outer injector body (1).

5. The injector in accordance with one of the preceding claims, **characterized by** webs (27) in the end region of he outer injector body (1).

6. The injector in accordance with one of the preceding claims, **characterized by** an elevated portion (28) in the base wall (6) of the outer injector body (1).

7. The injector in accordance with one of the preceding claims, **characterized in that** the cuff (13) is releasably connected to the inner injector body (2).

8. The injector in accordance with one of the claims 1 to 6, **characterized in that** the cuff (13) is formed in one piece with the inner injector body (2).

9. The injector in accordance with one of the claims 1 to 8, **characterized in that** one or more channels (15) are provided in the base wall (12) of the inner injector body (2) extend from the volume (24) of the inner injector body (2) to an annular space between the base wall (12) of the inner injector body (2) and the cuff (13).

10. The injector in accordance with claim 1 or 9, **characterized in that** the channels (15, 15') extend at least partially in a radial direction.

## Revendications

1. Injecteur de distribution d'une première et d'une seconde matière liquide ou pâteuse, en particulier d'un médicament, comprenant un corps extérieur d'injecteur (1), dans lequel un corps intérieur d'injecteur (2) est guidé de manière déplaçable longitudinalement, dans lequel un piston (3) est guidé de manière déplaçable longitudinalement, le corps extérieur d'injecteur comportant une partie enveloppe (5), une paroi de fond (6) et un bec de distribution, la paroi de fond (6) comportant en son centre un orifice de distribution (9), qui est relié à un conduit de distribution (10) dans le bec de distribution, le corps intérieur d'injecteur (2) comportant une paroi de fond (12), dans laquelle sont prévus un ou plusieurs conduits (15), et comportant une coupelle (13) s'appliquant de manière étanche contre la paroi intérieure (8) du corps extérieur d'injecteur (1), la seconde matière liquide ou pâteuse pouvant s'écouler autour de ladite coupelle quand celle-ci se trouve dans la position finale dans le corps extérieur d'injecteur (1), de telle sorte que, après la distribution de la première matière liquide ou pâteuse, la seconde matière liquide ou pâteuse peut être distribuée, **caractérisé en ce que** celle-ci est pressée dans l'orifice de distribution (9) et le conduit de distribution (10) en contournant la coupelle (13).

2. Injecteur selon la revendication 1, **caractérisé en ce que** le corps extérieur d'injecteur (1) comporte, dans sa zone d'extrémité, un ou plusieurs conduits (26).

3. Injecteur selon la revendication 1 ou 2, **caractérisé en ce que** la coupelle (13) comporte, sur sa face inférieure, une ou plusieurs rainures (26a).

4. Injecteur selon l'une des revendications précédentes, **caractérisé en ce que** la coupelle (13) est déformée de façon qu'un fluide peut circuler autour de celle-ci, quand elle se trouve dans la position finale dans le corps extérieur d'injecteur (1).

5. Injecteur selon l'une des revendications précédentes, **caractérisé par** des nervures (27) dans la zone d'extrémité du corps extérieur d'injecteur (1).

6. Injecteur selon l'une des revendications précédentes, **caractérisé par** une saillie (28) dans la paroi de fond (6) du corps extérieur d'injecteur (1).

7. Injecteur selon l'une des revendications précédentes, **caractérisé en ce que** la coupelle (13) est reliée de manière détachable au corps intérieur d'injecteur (2).

8. Injecteur selon l'une des revendications 1 à 6, **caractérisé en ce que** la coupelle (13) est réalisée d'un seul tenant avec le corps intérieur d'injecteur (2).

9. Injecteur selon l'une des revendications 1 à 8, **caractérisé en ce que** le ou les conduits (15) dans la paroi de fond (12) du corps intérieur d'injecteur (2) conduisent du volume (24) du corps intérieur d'injecteur (2) à un espace annulaire situé entre la paroi de fond (12) du corps intérieur d'injecteur (2) et la coupelle (13).

10. Injecteur selon la revendication 1 ou 9, **caractérisé en ce que** les conduits (15, 15') s'étendent au moins en partie dans la direction radiale.
